(19) Europäisches Patentamt
European Patent Office
Office européen des brevets

(11) Veröffentlichungsnummer: **0 270 972 B1**

# (12) EUROPÄISCHE PATENTSCHRIFT

(45) Veröffentlichungstag der Patentschrift: **09.09.92**

(51) Int. Cl.$^5$: **A61K 7/13**

(21) Anmeldenummer: **87117675.6**

(22) Anmeldetag: **30.11.87**

## (54) Oxidationshaarfärbemittel.

(30) Priorität: **08.12.86 DE 3641832**

(43) Veröffentlichungstag der Anmeldung:
**15.06.88 Patentblatt 88/24**

(45) Bekanntmachung des Hinweises auf die Patenterteilung:
**09.09.92 Patentblatt 92/37**

(84) Benannte Vertragsstaaten:
**AT BE CH DE ES FR GB GR IT LI LU NL SE**

(56) Entgegenhaltungen:
**EP-A- 0 195 361**
**EP-A- 0 226 197**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Konrad, Günther, Dr.**
**Feuerbachweg 12**
**W-4010 Hilden(DE)**
Erfinder: **Möller, Hinrich, Dr.**
**Schumannstr. 11**
**W-4019 Monheim(DE)**
Erfinder: **Lieske, Edgar**
**Hunsrückenstr. 40**
**W-4000 Düsseldorf(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 270 972 B1

**Beschreibung**

Gegenstand der Erfindung ist die Verwendung von N-(2,4-Dihydroxybenzyliden)-Aminoverbindungen als Oxidationsfarbstoffvorprodukte in Haarfärbemitteln. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluß von Oxidationsmitteln oder von Luftsauerstoff Farbstoffe ausbilden.

Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschte Farbnuancen in ausreichender Intensität ausbilden. Sie müssen ferner ein gutes Aufziehvermögen auf menschlichem Haar besitzen, ohne die Kopfhaut zu stark anzufärben. Die damit erzeugten Färbungen müssen eine hohe Stabilität gegen Abrieb, Wärme, Licht und die bei der Dauerwellung des Haars verwendeten Chemikalien aufweisen. Schließlich sollen die Oxidationshaarfarbstoffvorprodukte in toxikologischer und dermatologischer Hinsicht unbedenklich sein.

Es wurde gefunden, daß N-(2,4-Dihydroxybenzyliden)-Aminoverbindungen der Formel I

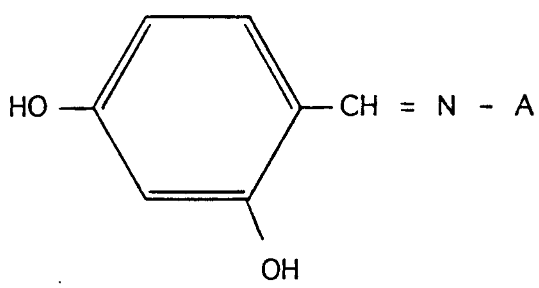

(I)

in der A eine Gruppe der Formeln II, III, IV, V oder VI

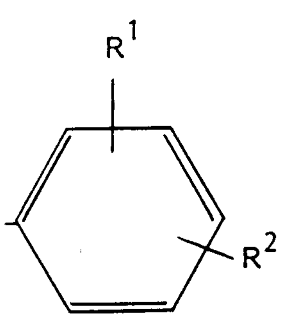

(II)

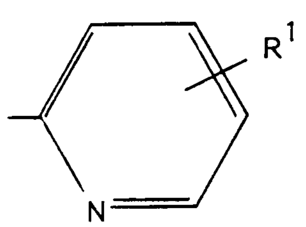

(III)

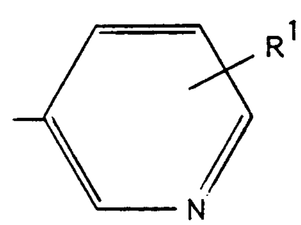

(IV)

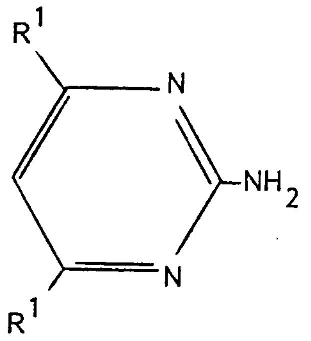

(V)

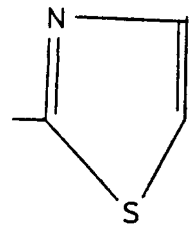

(VI)

darstellt, worin $R^1$ Wasserstoff oder eine Alkylgruppe mit 1 bis 4 C-Atomen und $R^2$ eine Alkylgruppe mit 1 bis 4 C-Atomen, eine Alkoxygruppe mit 1 bis 4 C-Atomen, eine COOH- oder eine $SO_3H$-Gruppe ist oder deren Salze als Oxidationsfarbstoffvorprodukte vom Kuppler-Typ gemeinsam mit üblichen Entwicklersubstanzen zur Herstellung von Oxidationshaarfärbemitteln geeignet sind und die genannten Anforderungen in hohem Maße erfüllen.

Die Gruppen $R^1$ und $R^2$ in Formel II können in beliebiger Position zueinander und zur Azomethingruppe angeordnet sein, auch in den Formeln III und IV kann $R^1$ in beliebiger Position am Pyridinkern stehen. Bevorzugt ist der Einsatz von Verbindungen der Formel I, in der A eine Gruppe der Formel II darstellt, worin $R^1$ Wasserstoff und $R^2$ eine $-OCH_3$, eine -COOH oder eine $-SO_3H$-Gruppe ist oder deren Salze.

Verbindungen der Formel I eignen sich auch als direktziehende Haarfarbstoffe und Haarfärbemittel mit einem Gehalt an direktziehenden Haarfarbstoffen der Formel I, in der A eine Gruppe der Formel II ist, sind bereits Gegenstand der deutschen Patentanmeldung P 35 45 245.5. Es wurde jedoch gefunden, daß die N-(2,4-Dihydroxybenzyliden)-Aminoverbindungen der Formel I auch Kupplereigenschaften aufweisen, d. h. daß sie mit bekannten Entwicklerkomponenten unter dem Einfluß von Oxidationsmitteln durch oxidative Kupplung besonders intensive, natürliche Oxidationshaarfärbungen mit besonders guten Echtheitseigenschaften ausbilden.

In die Haarfärbemittel werden erfindungsgemäß die N-(2,4-Dihydroxybenzyliden)-Aminoverbindungen der Formel I bevorzugt in freier Form eingesetzt, es können aber auch, wenn saure oder basische Gruppen enthalten sind, die entsprechenden Salze eingesetzt werden. Die Verbindungen mit -COOH- und $-SO_3H$-Gruppen können etwa in Form der Alkali- oder Ammoniumsalze, die Verbindungen der Formel I in der A eine Gruppe der Formel V ist auch in Form der Hydrochloride, Sulfate, Phosphate, Acetate, Propionate, Lactate oder Citrate eingesetzt werden.

Als Entwickiersubstanzen können erfindungsgemäß in den Haarfärbemitteln z.B. aromatische Amine mit einer oder mehreren weiteren $NH_2$-Gruppen, NHR-Gruppen, $NR_2$-Gruppen, wobei R eine Alkylgruppe mit 1 - 4 C-Atomen oder eine Hydroxyalkylgruppe oder eine Aminoalkylgruppe mit 2 - 4 C-Atomen darstellt, Aminophenole, Aminophenolether, Diaminopyridinderivate oder 2.4.5.6-Tetraaminopyrimidin und dessen Derivate wie z.B. 4.5-Diamino-2.6-bis-methylaminopyrimidin, 2.5-Diamino-4-diethylamino-6-methylaminopyrimidin, 2.4.5-Triamino-6-anilino-pyrimidin, 2.4.5-Triamino-6-morpholino-pyrimidin, 2.4.5-Triamino-6-(2-hydroxyethyl)-aminopyrimidin.

Besonders wertvolle, natürliche gelbe bis olivbraune Nuancen werden erhalten, wenn die N-(2,4-Dihydroxybenzyliden)-Aminoverbindungen der Formel I in Oxidationshaarfärbemitteln zusammen mit Entwicklersubstanzen vom Typ der aromatischen Diamine, insbesondere mit p-Phenylendiamin und dessen Derivaten enthalten sind.

Solche bevorzugten Entwicklersubstanzen sind z.B. p-Phenylendiamin, p-Toluylendiamin, N-Methyl-p-phenylendiamn, N,N-Dimethyl-p-phenylendiamin, N-Hydroxyethyl-p-phenylendiamin, N,N-Bis-(2-hydroxyethyl)-p-phenylendiamin, N-Ethyl-N-(2-hydroxyethyl)-p-phenylendiamin, N,N-Diethyl-2-methyl-p-phenylendiamin, 2-Chlor-p-phenylendiamin, 2,6-Dichlor-p-phenylendiamin, 2-Chlor-6-methyl-p-phenylendiamin, 2-Methoxy-p-phenylendiamin, 2.5-Diaminoanisol, 6-Methoxy-3-methyl-p-phenylendiamin, N-(2-Methoxyethyl)-p-phenylendiamin, N-(2-Hydroxypropyl)-p-phenylendiamin, N-Butyl-N-sulfobutyl-p-phenylendiamin, N-(p-Aminophenyl)-N',N'-bis-($\beta$-hydroxyethyl)-1.3-diaminopropan oder deren Salze mit anorganischen oder organischen Säuren.

Die Haarfärbemittel können erfindungsgemäß neben den N-(2,4-Dihydroxybenzyliden)-Aminoverbindungen der allgemeinen Formel I auch andere bekannte Kupplersubstanzen enthalten, die zur Modifizierung der Farbnuancen und zur Erzeugung natürlicher Farbtöne erforderlich sind. Solche üblichen Kupplerverbindungen sind z.B andere m-Phenylendiamine, z.B. 2.4-Diaminophenyl-2-hydroxyethylether, 2.4-Diaminoanisol oder Phenole, Resorcine, m-Aminophenole, Naphthole oder Pyrazolone.

Gegebenenfalls können auch direktziehende Farbstoffe zusätzlich zur weiteren Modifizierung der Farbnuancen eingesetzt werden. Solche direktziehenden Farbstoffe sind z.B. Nitrophenylendiamine, Nitroaminophenole, Anthrachinonfarbstoffe oder Indophenole.

Zu den Haarfärbemitteln werden erfindungsgemäß die N-(2,4-Dihydroxybenzyliden)-Aminoverbindungen der Formel I und die gegebenenfalls zusätzlich vorhandenen bekannten Kupplersubstanzen im allgemeinen in etwa molaren Mengen, bezogen auf die verwendeten Entwicklersubstanzen, eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklersubstanzen und Kupplersubstanzen in einem Molverhältnis von 1 : 0,5 bis 1 : 2 enthalten sein können.

Es ist nicht erforderlich, daß die N-(2,4-Dihydroxybenzyliden)-Aminoverbindungen der Formel I sowie die sonst in den Haarfärbemitteln vorhandenen Oxidationsfarbstoffvorprodukte oder direkt ziehenden Farbstoffe einheitliche chemische Verbindungen darstellen. Vielmehr können diese auch Gemische der erfindungsgemäß einzusetzenden Kuppler- oder Entwicklersubstanzen sein.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt am Haar gewtünscht wird. Als Oxidationsmittel kommen insbesondere Wasserstoffperoxid oder dessen Anlagerungsprodukte an Harnstoff, Melamin oder Natriumborat sowie Gemische aus derartigen Wasserstoffperoxidanlagerungsprodukten mit Kaliumperoxiddisulfat in Betracht.

Zur Herstellung der Haarfärbemittel werden erfindungsgemäß die Oxidationsfarbstoffvorprodukte in einen geeigneten kosmetischen Träger eingearbeitet. Solche Träger sind z.B. Cremes, Emulsionen, Gele oder auch tensidhaltige, schäumende Lösungen, z.B. Shampoos oder andere Zubereitungen, die für die Anwendung auf dem Haar geeignet sind. Übliche Bestandteile solcher kosmetischer Zubereitungen sind z.B. Netz- und Emulgiermittel wie anionische, nichtionische oder ampholytische Tenside, z.B. Fettalkoholsulfate, Alkansulfonate, alpha-Olefinsulfonate, Fettalkoholpolyglykolethersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z.B. Methyl- oder Hydroxyethylcellulose, Stärke, Fettalkohole, Paraffinöle, Fettsäuren, ferner Parfümöle und haarpflegende Zusätze, wie z.B. wasserlösliche kationische Polymere, Proteinderivate, Pantothensäure und Cholesterin.

Die Bestandteile der kosmetischen Träger werden zur Herstellung der Haarfärbemittel in für diese Zwecke üblichen Mengen eingesetzt, z.B. werden Emulgiermittel in Konzentrationen von 0,5 - 30 Gew.-% und Verdickungsmittel in Konzentrationen von 0,1 - 25 Gew.-% des gesamten Färbemittels eingesetzt. Die Oxidationsfarbstoffvorprodukte werden in Mengen von 0,2 - 5 Gew.-%, vorzugsweise 1 - 3 Gew.-% des gesamten Färbemittels in den Träger eingemischt. Der Gehalt an N-(2,4-Dihydroxybenzyliden)-Aminoverbindungen der Formel I kann in den erfindungsgemäßen Haarfärbemitteln etwa 0,05 - 10 Millimol pro 100 g des Haarfärbemittels betragen.

Die Anwendung der Haarfärbemittel kann erfindungsgemäß, unabhängig von der Art der kosmetischen Zubereitung, z.B. als Creme, Gel oder Shampoo im schwach sauren, neutralen oder alkalischen Milieu erfolgen. Bevorzugt ist die Anwendung der Haarfärbemittel in einem pH-Bereich von 8 - 10. Die Anwendungstemperaturen können in einem Bereich zwischen 15 ° C und 40 ° C liegen. Nach einer Einwirkungszeit von ca. 30 Minuten wird das Haarfärbemittel durch Ausspülen von dem zu färbenden Haar entfernt. Danach wird das Haar mit einem milden Shampoo nachgewaschen und getrocknet. Das Nachwaschen mit einem Shampoo entfällt, wenn ein stark tensidhaltige Träger, z.B. ein Färbeshampoo verwendet wurde.

Die nachfolgenden Beispiele sollen den Erfindungsgegenstand näher erläutern.

## Beispiele

### I. Herstellung von N-(2,4-Dihydroxybenzyliden)-Aminoverbindungen der Formel I

Die Produkte 1, 2 und 6 bis 10 der Tabelle I wurden durch Vereinigung ethanolischer Lösungen molarer Mengen von 2,4-Dihydroxybenzaldehyd und

(1) p-Anisidin
(2) m-Anisidin
(6) 2,6-Dimethylanisidin
(7) 2-Aminothiazol

(8) 2-Aminopyridin
(9) 3-Aminopyridin
(10) 2,5-Diamino-4,6-dimethylpyrimidin

hergestellt. Die Azomethine fielen nach einigen Stunden bei 20 °C aus der Lösung aus und wurden abfiltriert und mit Ethanol gewaschen und getrocknet.

Die Produkte 3, 4 und 5 der Tabelle I wurden durch Vereinigung ethanolischer Lösungen molarer Mengen von 2,4-Dihydroxybenzaldehyd und

(3) Anthranilsäure
(4) p-Aminobenzoesäure und
(5) Sulfanilsäure

hergestellt. Das Reaktionsgemisch wurde nach dem Zusammengeben 5 Stunden erhitzt. Nach dem Abkühlen wurden die ausgefallenen Azomethine abfiltriert, mit Ethanol gewaschen und getrocknet.

Tabelle I

| | Azomethin | Schmelzpunkt | |
|---|---|---|---|
| 1 | N-(2,4-Dihydroxybenzyliden)-p-anisidin | ab 90 °C | gelbbraune Kristalle |
| 2 | N-(2,4-Dihydroxybenzyliden)-m-anisidin | 151 °C | graugelbe Kristalle |
| 3 | N-(2,4-Dihydroxybenzyliden)-anthranilsäure | über 250 °C | gelbe Kristalle |
| 4 | N-(2,4-Dihydroxybenzyliden)-p-aminobenzoesäure | 218 °C | gelbe Kristalle |
| 5 | N-(2,4-Dihydroxybenzyliden)-sulfanilsäure | über 250 °C | gelborange Kristalle |
| 6 | N-(2,4-Dihydroxybenzyliden)-2,6-dimethylanilin | 145 °C | hellbraunes Pulver |
| 7 | 2-(2,4-Dihydroxybenzylidenamino)-thiazol | ab 80 °C | gelbbraune Kristalle |
| 8 | 2-(2,4-Dihydroxybenzylidenamino)-pyridin | über 270 °C | gelbe Kristalle |
| 9 | 3-(2,4-Dihydroxybenzylidenamino)-pyridin | ab 180 °C | gelbe Kristalle |
| 10 | 2-Amino-5-(2,4-dihydroxybenzylidenamino)-4,6-dimethylpyrimidin | 205 - 206 °C unter Zers. | gelbe Kristalle |

Anwendungstechnische Prüfungen

Es wurden erfindungsgemäße Haarfärbemittel in Form einer Haarfärbe-Cremeemulsion der folgenden Zusammensetzung hergestellt:

| | |
|---|---|
| Fettalkohol $C_{12-14}$ | 10 g |
| Fettalkohol $C_{12-14}$ + 2 EO-sulfat, Na-Salz, 28 %ig | 25 g |
| Wasser | 60 g |
| Kupplersubstanz (Azomethin aus Tab. I) | 0,0075 Mol |
| p-Toluylendiamin | 0,0075 Mol |
| $Na_2SO_3$ (Inhibitor) | 1,0 g |
| konzentrierte Ammoniak-Lösung | bis pH = 9,5 |
| Wasser | ad 100 g |

Die Bestandteile wurden der Reihe nach miteinander vermischt. Nach Zugabe der Oxidationsfärbemittelvorprodukte und des Inhibitors wurde zunächst mit konzentrierter Ammoniak-Lösung der pH-Wert der Emulsion auf 9,5 eingestellt, dann wurde mit Wasser auf 100 g aufgefüllt.

Die oxidative Entwicklung der Färbung wurde mit 3 %iger Wasserstoffperoxidlösung als Oxidationsmittel durchgeführt. Hierzu wurden 100 g der Emulsion mit 50 g Wasserstoffperoxidlösung (3-%ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 27 °C belassen. Nach Beendigung des Färbeprozesses wurde das Haar gespült, mit einem üblichen Haarwaschmittel ausgewaschen und anschließend getrocknet.

Die Ergebnisse der Färbeversuche sind Tabelle II zu entnehmen.

Tabelle II

| Azomethin aus Tabelle I | Nuance der gefärbten Haarsträhne |
|---|---|
| 1 | olivbraun |
| 2 | olivbraun |
| 3 | olivbraun |
| 4 | olivgelb |
| 5 | olivbraun |
| 6 | olivbraun |
| 7 | olivbraun |
| 8 | gelbbraun |
| 9 | gelbbraun |
| 10 | olivbraun |

**Patentansprüche**

**1.** Verwendung Von N-(2,4-Dihydroxybenzyliden)-Aminoverbindrnngen der Formel I

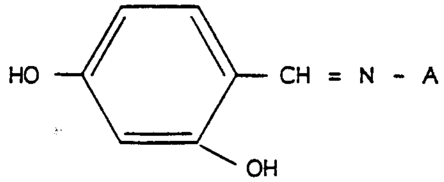 (I)

in der A eine Gruppe der Formeln II, III, IV, V oder VI

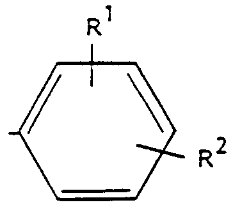

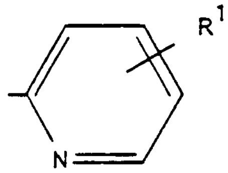

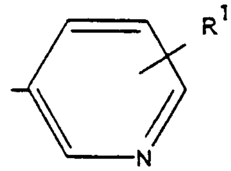

(II) (III) (IV)

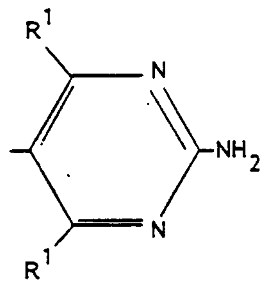

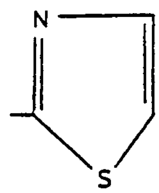

(V) (VI)

darstellt, worin $R^1$ Wasserstoff oder eine Alkylgruppe mit 1 bis 4 C-Atomen und $R^2$ eine Alkylgruppe mit 1 bis 4 C-Atomen, eine Alkoxygruppe mit 1 bis 4 C-Atomen, eine COOH- oder eine $SO_3H$-Gruppe ist oder deren Salze als Oxidationsfarbstoffvorprodukte vom Kuppler-Typ gemeinsam mit üblichen Entwicklersubstanzen zur Herstellung von Oxidationshaarfärbemitteln.

**2.** Verwendung nach Anspruch 1, dadurch gekennzeichnet, daß in den Kupplersubstanzen der Formel I A eine Gruppe der Formel II darstellt, worin $R^1$ Wasserstoff und $R^2$ eine $-OCH_3$-, eine -COOH- oder eine $-SO_3H$-Gruppe ist.

**3.** Verwendung nach Anspruch 1 oder 2, dadurch gekennzeichnet, daß die Entwicklersubstanzen p-Phenylendiamin oder dessen Derivate sind.

**4.** Verwendung nach Anspruch 1 - 3, dadurch gekennzeichnet, daß zusätzlich weitere, bekannte Kupplersubstanzen eingesetzt werden, daß Entwicklersubstanzen und Kupplersubstanzen im Molverhältnis 1 : 0,5 bis 1 : 2 eingesetzt werden, daß Oxidationsfarbstoffvorprodukte in einer Menge von 0,2 - 5,0 Gew.-% des Haarfärbemittels und N-(2,4-Dihydroxybenzyliden)-Aminoverbindungen der Formel I in einer Menge von 0,05 - 10 Millimol pro 100 g des Haarfärbemittels eingesetzt werden.

## Claims

**1.** The use of N-(2,4-dihydroxybenzylidene)-amino compounds corresponding to formula I

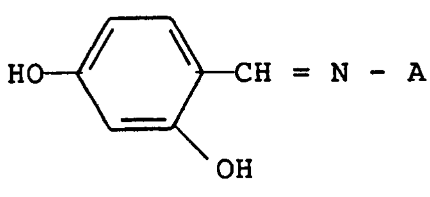

(I)

in which A is a group corresponding to formula II, III, IV, V or VI

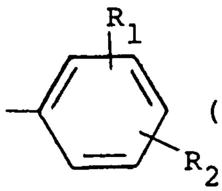 (II) 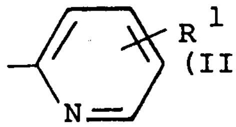 (III) 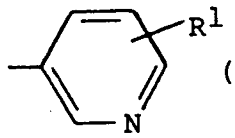 (IV)

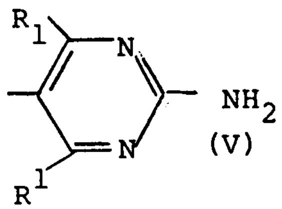 (V) 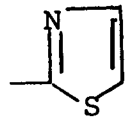 (VI)

in which

$R^1$ is hydrogen or a $C_{1-4}$ alkyl group and $R^2$ is a $C_{1-4}$ alkyl group, a $C_{1-4}$ alkoxy group, a COOH or an $SO_3H$ group, or salts thereof as oxidation dye precursors of the coupler type, together with typical developer components, for the production of oxidation hair-dyeing preparations.

**2.** The use claimed in claim 1, characterized in that, in the couplers corresponding to formula I, A is a group of formula II in which $R^1$ is hydrogen and $R^2$ is an $-OCH_3$, a -COOH or an $-SO_3H$ group.

**3.** The use claimed in claim 1 or 2, characterized in that the developers are p-phenylenediamine or derivatives thereof.

**4.** The use claimed in claims 1 to 3, characterized in that other known couplers are additionally used, in that developers and couplers are present in a molar ratio of from 1:0.5 to 1:2 and in that oxidation dye precursors are used in a quantity of 0.2 to 5.0% by weight, based on the hair-dyeing preparation, and N-(2,4-dihydroxybenzylidene)-amino compounds of formula I are used in a quantity of 0.05 to 10 millimol per 100 g of the hair-dyeing preparation.

**Revendications**

**1.** Utilisation de composés aminés de N-(2,4-dihydroxybenzylidène) de formule I :

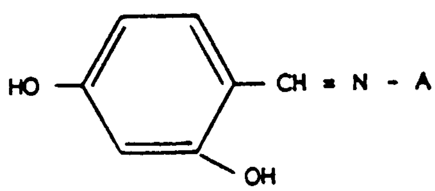 (I)

dans laquelle A représente un groupe des formules II, III, IV, V ou VI,

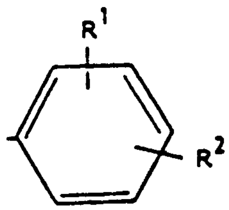

(II)

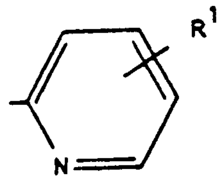

(III)

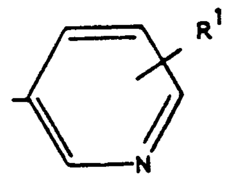

(IV)

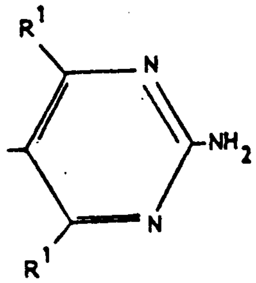

(V)

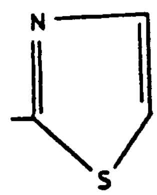

(VI)

dans lesquels $R^1$ est de l'hydrogène ou un groupe alkyl avec de 1 à 4 atomes de C et $R^2$ un groupe alkyl avec de 1 à 4 atomes de C, un groupe alcoxy avec de 1 à 4 atomes de C, un groupe COOH- ou un groupe $SO_3H$- ou leurs sels comme produits préliminaires de colorants d'oxydation du type coupleur en commun avec des substances de développements usuelles pour la production d'agents de coloration des cheveux avec oxydation.

**2.** Utilisation selon la revendication 1, caractérisée en ce que dans les substances d'accouplement de formule I, A représente un groupe de formule II, dans lequel $R^1$ est de l'hydrogène et $R^2$ est un groupe $-OCH_3$-, un groupe -COOH- ou un groupe $-SO_3H$-.

**3.** Utilisation selon les revendications 1 ou 2, caractérisée en ce que les substances de développement sont de la p-phénylènediamine ou ses dérivés.

**4.** Utilisation selon les revendications 1 à 3, caractérisée en ce qu on met en oeuvre d'autres substances d'accouplement supplémentaires connues,

- en ce que substances de développement et substances de couplage sont utilisées dans le rapport molaire de 1:0,5 à 1:2,
- en ce que les produits préliminaires de teinture par oxydation sont utilisés dans une quantité de 0,2 à 5 % en poids de colorant pour cheveux et les composés aminés de N-(2,4-dihydroxybenzylidène) de la formule I dans une quantité de 0,05 à 10 millimol pour 100 g de colorant pour cheveux.